# FASCICULE DE BREVET EUROPEEN

(11) **EP 1 150 999 B1**
(45) Date de publication et mention de la délivrance du brevet: **21.06.2006**
(21) Numéro de dépôt: 00901297.2
(22) Date de dépôt: 07.02.2000
(51) Int. Cl.: C07K 7/64, A61K 38/13

(54) **DERIVES DE CYCLOSPORINE ET PROCEDE DE PREPARATION DESDITS DERIVES**
CYCLOSPORIN-DERIVATE UND VERFAHREN ZU DEREN HERSTELLUNG
CYCLOSPORIN DERIVATIVES AND METHOD FOR THE PRODUCTION OF SAID DERIVATIVES

(30) Priorité: 05.02.1999 CH 22099
(43) Date de publication de la demande: 07.11.2001
(73) Titulaire: DEBIOPHARM S.A., 1000 Lausanne 9 (CH)
(72) Inventeur: MUTTER, Manfred, CH-1028 Préverenges (CH); WENGER, Roland, M., CH-4125 Riehen (CH); GUICHOU, Jean-François Université de Lausanne, CH-1015 Lausanne (CH); KELLER, Michael c/o A. Miller Imperial College, London SW9 21Y (GB); RUCKLE, Thomas Université de Lausanne, CH-1015 Lausanne (CH); WOEHR, Torsten, CH-8037 Zürich (CH)
(86) Numéro de dépôt international: PCT/IB2000/000133
(87) Numéro de publication internationale: WO 2000/046239

(56) Documents cités:
- EP-A- 0 034 567
- EP-A- 0 300 785
- WO-A-00/01715
- GB-A- 2 205 317
- CHEMICAL ABSTRACTS, vol. 127, no. 95603, 5 janvier 1998 (1998-01-05) Columbus, Ohio, US; abstract no. 366197, KO SOO Y. AND WENGER ROLAND M.: "solide phase total synthesis of cyclosporin analogs" XP002133829 & HELV.CHEM.ACTA, vol. 80, no. 3, 1997, pages 695-705,

## Description

La présente invention concerne des dérivés de cyclosporine, la séquence peptidique desdits dérivés comprenant au moins un acide aminé non naturel de type pseudo-proline. Elle concerne également un procédé de préparation desdits dérivés.

Les cylosporines constituent une famille de métabolites secondaires obtenus par fermentation. Ces substances possèdent des propriétés biologiques remarquables. Parmi celles-ci, on peut citer l'immunosuppression, l'induction de la prolifération des nerfs dans les maladies neuro-dégénératives ou encore l'arrêt de la réplication du virus VIH-1. A ce jour, une trentaine de cyclosporines ont été isolées de source naturelle. La plus connue, par son utilisation lors de transplantation d'organe, est la cyclosporine A (CsA). Par la suite, il a été trouvé que cette même cyclosporine A pouvait ouvrir de nouvelles voies dans le traitement du SIDA en inhibant l'activation des cellules CD4⁺.

Les cyclosporines sont constituées d'une séquence peptidique cyclique complexe de onze acides aminés, certains d'entre eux étant des acides aminés non-naturels fréquemment méthylés sur l'azote. Ces substance possèdent un fort caractère hydrophobe qui rend compliqué leur admimistration au sein d'un milieu physiologique.

A ce jour, il est toujours nécessaire d'entreprendre des modifications de structure de façon à améliorer l'activité biologique et/ou les propriétés physico-chimiques des cydosporines existantes, qu'elles soient d'origine naturelle ou synthétique.

Ainsi, la demande de brevet GB 2 205 317 décrit des analogues de la Cyclosporine A qui contiennent des acides aminés modifiées en position 9 et qui présentent des activités immunosuppressives.

S. Y. Ko et al décrivent dans Helvetica Chimica Acta, 1997, 80, 695-705 une série d'analogues de la Cyclosporine A et ont étudié les effets de modifier les acides aminés en positions 4, 6 7 et 8 sur une activité immunosupressive.

La demande de brevet EP 0 300 785 décrit des analogues de la Cyclosporine A dans lesquels l'acide aminé en position 1 a été modifié et utilisables en tant qu'agents immunosuppresseurs.

La demande de brevet EP 0 034 567 décrit une méthode de synthèse totale d'analogues de la Cyclosporine A.

A cet effet, l'un des buts de la présente invention est de mettre à disposition des dérivés de cyclosporine d'origine naturelle ou synthétique, dérivés dont la spécificité pharmacologique a été améliorée, de préférence en faveur de l'inhibition de l'activation des cellules CD4⁺ afin d'arrêter la réplication du virus VIH-1.

Un autre des buts de la présente invention est de mettre à disposition des dérivés de cyclosporine d'origine naturelle ou synthétique, dérivés dont les propriétés physiques ont été modifiées de façon à leur conférer un certain caractère hydrophile afin d'accroître leur solubilisation dans un milieu physiologique et de faciliter ainsi leur administration.

Ainsi, la présente invention a pour objet des dérivés de cyclosporine d'origine naturelle ou non, l'enchaînement peptidique desdits dérivés comprenant au moins un résidu d'acide aminé non naturel de formule générale I suivante: 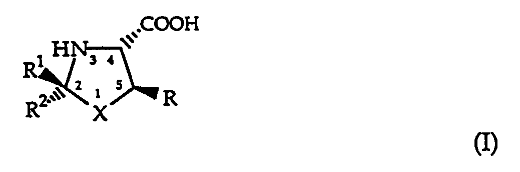 dans laquelle,
X représente un oxygène ou un soufre;
R représente un hydrogène ou un alkyle ayant de 1 à 6 atomes de carbone, de préférence un méthyle;
R₁ et R₂ représentent, indépendamment l'un de l'autre, un hydrogène, un alkyle ayant de 1 à 6 atomes de carbone, linéaire ou ramifié, substitué ou non, un alkylène ayant de 1 à 6 atomes de carbone, un aryle non substitué, tel que le phényl, un aryle substitué, tel que le p-carbométhoxy-phényl ou le p-méthoxy-phényl, un hétéro-aryle substitué ou non.

R¹ et R² peuvent également représenter un résidu d'un polymère hydrosoluble relié éventuellement à un groupe d'espacement ou "spacer". Des exemples appropriés d'un tel polymère incluent des oxydes de polyalkylène (PAO) tels les polyéthylène glycols, des alcools polyvinyliques, des polymères à base de carbohydrate. De préférence, le polymère hydrosoluble est un oxyde de polyalkylène tel qu'un polyéthylène glycol. Le groupe d'espacement peut être un alkyle ayant de 1 à 6 atomes de carbone, un aryle, tel que le phényl, un hétéro-aryle, chacun portant un groupe fonctionnel permettant l'ancrage du polymère. Lorsque le polymère est un polyéthylène glycol, alors le groupe d'espacement préféré est le p-carboxy-phénylène.

Le nom générique de "pseudo-proline" a été donné dans la présente demande à l'acide aminé non naturel de formule générale I et les abréviations Ser(Ψ^{R1,R2}pro), Thr(Ψ^{R1,R2}pro) ou Cys(Ψ^{R1,R2}pro) indiquent que, dans la formule générale I, les symboles (X, R) représentent respectivement (O, H), (O, Me) et (S, H) et que l'acide aminé dérive respectivement de la serine, de la thréonine et de la cystéine.

De préférence, les dérivés de cyclosporine de la présente invention dérivent de cyclosporines, naturelles ou synthétiques, dont l'enchaînement peptidique contient au moins l'un des acides aminés suivants, sous la configuration d ou 1: sérine, thréonine ou cystéine. Dans la séquence peptidique des dérivés de cyclosporine de la présent invention, au moins l'un quelconque des acides aminés sérine, thréonine ou cystéine, sous la configuration d ou 1, des cyclosporines de référence a été remplacé par l'acide aminé non naturel de formule générale I.

Au vu de la complexité de l'enchaînement peptidique des cyclosporines, toute modification chimique de leurs structures s'avère rapidement compliqué. Une synthèse totale se trouve alors inappropriée.

A cet effet, un autre des buts de la présente invention est alors de mettre à disposition un procédé de préparation de ces dérivés de cyclosporine qui soit le plus simple possible en mettant en oeuvre des produits, cyclosporines et réactifs, facilement accessibles.

Ainsi, la présente invention a également pour objet un procédé de préparation des dérivés de cyclosporine dont l'enchaînement peptidique comprend au moins l'un des acides aminés sérine, thréonine ou cystéine par N,O-acétalisation d'au moins un des trois acides aminés précédent en mettant en contact la cyclosporine avec un composé de formule II suivante: 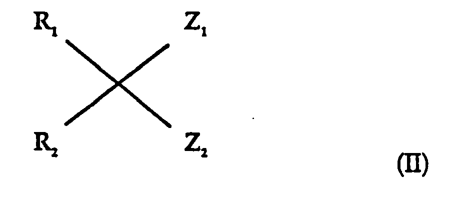 dans laquelle
Z₁ et Z₂ représentent, indépendamment l'un de l'autre, un halogène, un hydroxy, un alkoxy, un thiol; ou bien
Z₁ et Z₂ représentent ensemble, un oxygène d'un carbonyle ou un soufre d'une thione; et
R₁ et R₂ ayant la même définition que donnée précédemment.

De préférence, le composé de formule II est un acétal ou un thio-acétal.

Les propriétés, les avantages des dérivés de cyclosporine de la présente invention, ainsi que la manière de mettre en oeuvre le procédé de préparation de ces dérivés apparaîtront avec les exemples de réalisations présentés ci-après et à l'aide du dessin dans lequel
- la Fig. 1 représente le schéma de synthèse d'un dérivé de cyclosporine;
- la Fig. 2 représente le schéma de synthèse d'un intermédiaire de la préparation du dérivé de la Fig.1;
- la Fig. 3 représente les enregistrements de chromatogrammes HPLC au cours du temps d'un essai d'hydrolyse d'un dérivé de cyclosporine;
- la Fig. 4 est une courbe montrant l'évolution au cours du temps de la concentration des produits du même essai d'hydrolyse; et
- la Fig 5 représente une courbe de cinétique d'inhibition de l'activité cis/trans isomérase de la cyclophiline A du thymus de veau par un dérivé de cyclosporine.

Trois cyclosporines ont servi de référence pour préparer les dérivés selon l'invention. Deux de ces cyclosporines sont d'origine naturelle. Ce sont la cyclosporine A (CsA) et la cyclosporine C (CsC). La troisième cyclosporine, à savoir la [D-Ser⁸]cyclosporine A, est obtenue par fermentation en incorporant l'acide aminé D-sérine selon la procédure décrite par Traber et al. dans *The journal of Antibiotics, 1989.*

Deux séries d'expériences ont été réalisées selon la nature des dérivés de cyclosporine préparés. La première série d'expériences a été orientée vers la modification des propriétés physiques des cyclosporines, leur conférant en particulier un caractère hydrophile. La deuxième série a été orientée vers l'amélioration de leurs propriétés biologiques.

A ce propos et selon des études de structure-activité bien établies, on sait que la partie peptidique continue de la cyclosporine A constituée par les acides aminés situés en position 10 à 11,1 à 3 (système de numérotation prenant pour position 1 l'acide aminé MeBmt) se lie à la cyclophiline (CyP), protéine ayant une activité peptidylprolyl cis-trans isomérase. La partie peptidique libre se lie ensuite à la calcineurine (Cn) et le complexe ainsi formé [(CsA-CyP)-Cn] est responsable de l'immunosuppression en inhibant la transcription des gènes essentiels des cytokines. La cyclosporine C se différencie, en ce qui concerne sa structure, de la cyclosporine A par l'acide aminé en position 2, à savoir Ser au lieu de Abu. En revanche, son mode d'action est similaire.

### 1. Préparation des dérivés de la cyclosporine A, à savoir [5-L-Thr(Ψ^{R1,R2}pro)]CsA de formule générale III suivante:

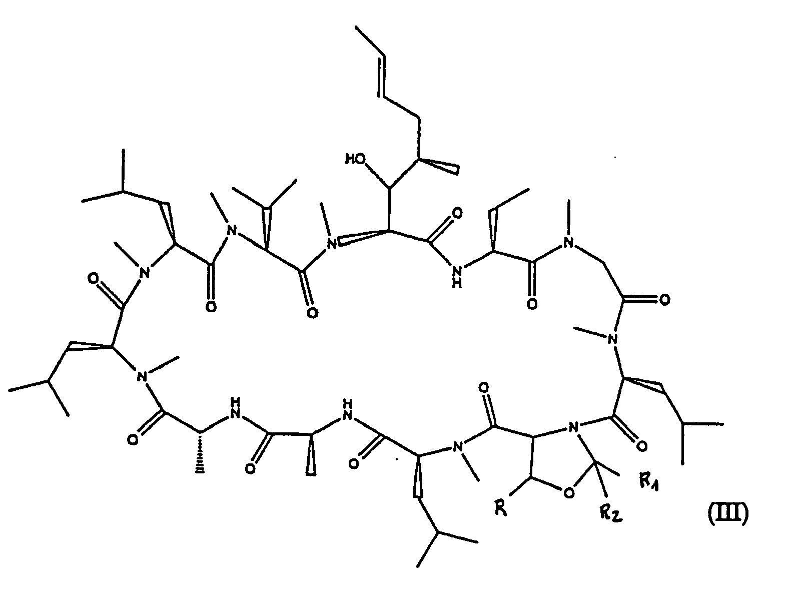

Dans les dérivés de la cyclosporine A de formule générale III, la pseudo-proline L-Thr(Ψ^{R1,R2} pro) occupe la position 5, se substituant ainsi à la valine de la cyclosporine A.

Pour se faire, le cycle de la cyclosporine A est ouvert par clivage de la liaison peptidique 4-5. La liaison peptidique 7-8 est à son tour clivée. Après des étapes de protection et d'activation, le dipeptide Fmoc-NMeLeu-L-Thr(Ψ^{R1,R2} pro)-OH, préparé préalablement, est lié à l'acide aminé Ala en position 7, puis le cycle peptidique est refermé, conduisant ainsi aux dérivés [5-L-Thr(Ψ^{R1,R2} pro)]CsA de la cyclosporine A.

Les dérivés de formule IIIa et IIIb ont été préparé en faisant réagir le dipeptide Fmoc-NMeLeu-L- Thr(Ψ^{R1,R2} pro)-OH approprié.

| **Dérivé** | **R**_{**1**} | **R**_{**2**} |
|---|---|---|
| IIIa | H | MeO-PEG 750-NHCO-phényl- |
| IIIb | Me | Me |

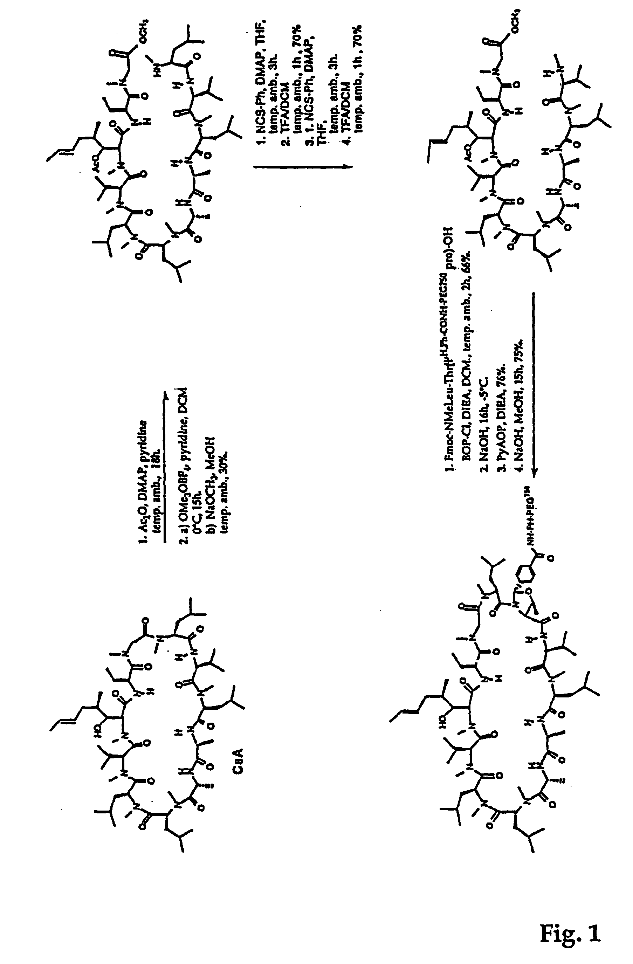

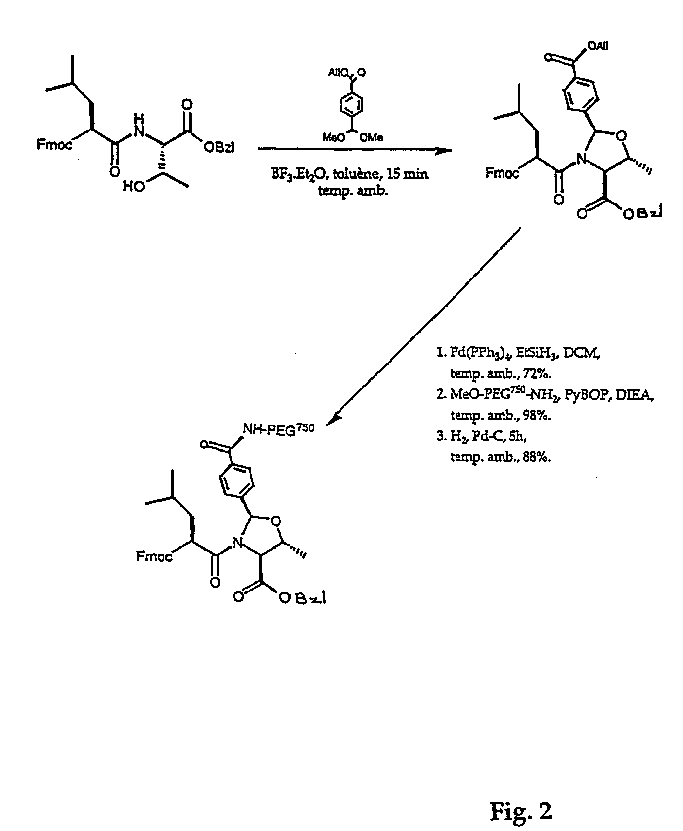

Le schéma de synthèse du dérivé IIIa, ainsi que selui de l'un de ses intermédiaires de synthèse, le dipepetide Fmoc-NMeLeu-L-Thr(Ψ^{MeO-PEG750-NHCO-phényl-H} pro)-OH, sont présentés en détail aux figures 1 et 2. Il apparait qu'un tel procédé mettant en jeu l'ouverture de la cyclosporine A, insertion d'un peptide contenant la pseudo-proline appropriée puis fermeture du cycle, s'il conduit bien à l'obtention des dérivées de la présente invention, ne peut pas, du fait de sa complixité, permettre la préparation d'un grand nombre de dérivés et ceci à grande échelle.

Nous présentons ci-après un mode de réalisation du procédé de préparation de la présente invention de dérivés de cyclosporine, en utilisant, en tant que cyclosporine de départ, des cyclosporines dont l'enchainement peptidique comprend au moins l'un des acides aminés sérine, thréonine ou cystéine.

En une seule étape mettant en jeu une réaction de N,O-acétalisation d'au moins un des trois acides aminés précédents à l'aide d'un composé approprié de formule II précédente, on obtient un dérivé de cyclosporine dans lequel, la pseudo-proline s'est substituée à l'un des acides aminés sérine, thréonine ou cystéine de la cyclosporine de départ.

### 2. Préparation des dérivés L-Thr(Ψ^{R1,R2}pro)]CsC de la cyclosporine C de formule générale IV suivante:

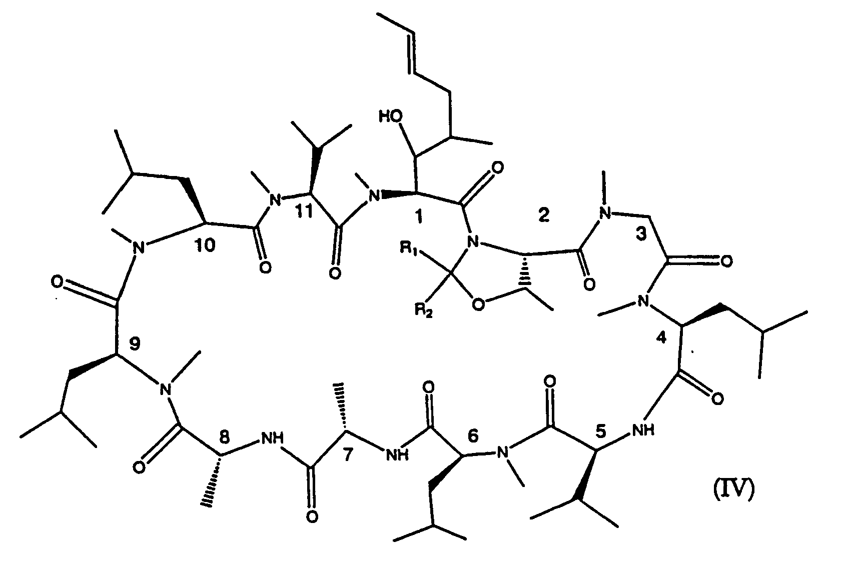

Les dérivés IVa à IVh ont été préparés selon la procédure générale suivante.

Un mélange comprenant de la cyclosporine C (CsC) anhydre (50 mg, 41 µMol), du diméthylacétal R₁R₂C(OMe)₂ (205 µMol, 5 eq) et le sel de pyridine de l'acide p-toluène sulfonique (4.0 mg, 0.4 eq, PPTS) dans du toluène anhydre (4ml) est porté au reflux. Une fois la réaction terminée, la phase organique est lavée par Na₂CO₃ (10%, 2x5 ml) et de l'eau (2x5 ml) et est séchée sur du sulfate de magnésium. La phase organique est concentrée sous pression réduite pour donner une huile. Le produit brut est dissous dans 2 ml d'un mélange acétonitrile/eau (1:1/v/v) et est purifié par HPLC en phase inverse (C₁₈, 60-100% B, 40 min). La lyophilisation conduit à l'obtention d'une poudre blanche du dérivé de cyclosporine C.

| **Dérivé** | **R**_{**1**} | **R**_{**2**} | **Temps de réaction (min)** | **Rdt (%)** | **Masse (calc.) trouvé m/z** |
|---|---|---|---|---|---|
| IVa | H | Ph- | 45 | 74 | (1306,7) 1306,7 |
| IVb | H | Ph-Ph- | 30 | 89 | (1382,8) 1383,8 |
| IVc | H | CH₂=CH- | 60 | 75 | (1256,7) 1257,7 |
| IVd | H | *p*-CO₂Me-Ph- | 120 | 55 | (1364,7) 1364,7 |
| IVe | H | *p*-OMe-Ph- | 60 | 90 | (1336,2) 1337,2 |
| IVf | H | *p*-AllOOC-Ph- | 50 | 95 | (1390,7) 1391 |
| IVg | H | *p*-HOOC-PhCH(OMe), | 50^{*d*} | 75 | (1350,7) 1351 |
| IVh | H | PEG⁸⁵⁰-CH- | 240 | 20 | (≈1851)≈1851^{e} |

### 3. Préparation des dérivés D-Ser⁸(Ψ^{R1,R2}pro)]CsA de la D-Ser⁸-cyclosporine A de formule générale V suivante:

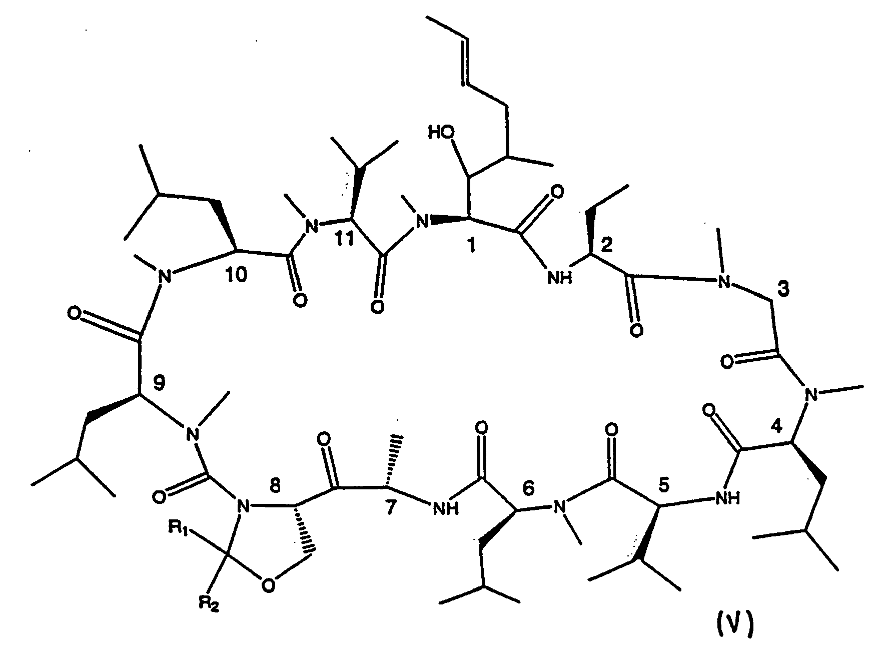

Les dérivés Va à Ve ont été préparés selon la procédure générale suivante.

Un mélange contenant de la cyclosporine D-Ser⁸-CsA (séchée) (1 éq.), du diméthylacétal R₁R₂C(OCH₃)₂ (10 éq.), du PPTS (pyridinium de l'acide para-toluène sulfonique) (0,4 éq.) et du DMSO anhydre (0,016 M) est chauffé à 100°. Le milieu réactionnel est versé dans 150 ml de AcOEt. La phase organique est lavée successivement avec une solution de NaHCO₃ saturée (3 fois) et une solution de NaCl saturée (1 fois), puis séchée sur Na₂SO₄ et concentrée. Le produit brut est purifié par chromatographie sur gel de silice (Acétone/Hexane 4/6), pour donner une poudre blanche.

| **Dérivé** | **R**_{**1**} | **R**_{**2**} | **Temps de réaction** | **Rdt en %** | **Rf (Acétone/ Hexane) (4/6)** | **HPLC en minute** | **Masse ESI-MS** |
|---|---|---|---|---|---|---|---|
| Va | CH_{**3**} | CH_{**3**} | 3 h | 58 | 0,25 | 17,98 | 1244/1276 /1293 |
| Vb | -CH₂OAc | H | 30 h | 74 | 0,32 | 18,65 | 1334/1351 |
| Vc | -(CH₂)-NH-Fmoc | H | 2 h | 70 | 0,25 | 17,96 | 1509/1526 |
| Vd | -Ph | H | 3 h | 72 | 0,50 | 19,16 | 1306/1323 /1328 |
| Ve | -p-Ph-CH₂-NH-Aloc | H | 20 mn | 67 | 0,54 | 19,42 | 1419/1436 |

### 4. Propriétés physiques des dérivés de cyclosporine de la présente invention.

### 4.1. Obtention de prémédicaments

Il a été trouvé, de manière surprenante, que l'introduction d'une pseudo-proline au sein de l'enchaînement d'une cyclosporine permettait d'obtenir un prémédicament ou "prodrug" de cette même cyclosporine.

En effet, la stabilité chimique des dérivés de la présente invention face, en particulier, à des conditions d'hydrolyse acide, a été étudiée en fonction de la nature des groupes portés en position para de noyaux phényl constituant les substituant R₁ ou R₂. Des groupes électro-attracteur stabilisent le cycle oxazolidine de la pseudo-proline. En revanche, des groupes électrodonneurs tel que le groupe méthoxy rendent la pseudo-proline très sensible aux environnements acide et, par réaction réversible, le cycle oxazolidine s'ouvre, libérant ainsi la sérine ou la thréonine de la cyclosporine de départ.

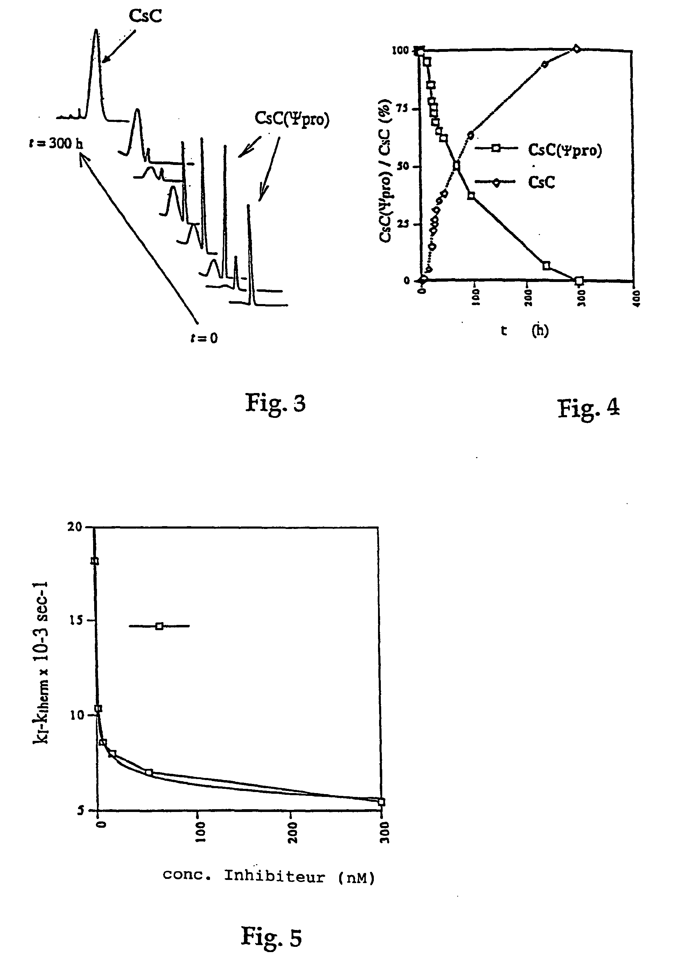

Par exemple, le dérivé IVd, obtenu à partir de la cyclosporine C, a été placé dans des conditions physiologiques telles que celles trouvées dans l'appareil digestif (pH 1, THF/HCl). Comme le montre les figures 3 et 4, en 300 heures, la cyclosporine a été entièrement reconstituée.

### 4.2. Obtention de dérivés hydrophiles

L'accrochage d'un polymère hautement hydrosoluble comme peut l'être le polyéthylène glycol dans les dérivés IIIb et IVh a supprimé le caractère hydrophobe des cyclosporines de départ, respectivement les cyclosporines AetC.

### 5. Activités biologiques des dérivés de cyclosporine de la présente invention, effet inhibiteur vis-à-vis de la cyclophiline A du thymus de veau.

Le test de binding décrit par Fisher et al. dans *Biomed. Biochim. Acta, 1984,* pour les cis/trans isomérases a été appliqué à la cyclophiline du thymus de veau (3.8nm), en prenant comme référence le binding de la cyclosporine A. Les rapports IC₅₀/IC_{50CSA}. sont rassemblés dans le tableau ci-après.

| **Dérivés** | IIIb | IVa | IVb | IVc | IVd | IVe | IVf | IVg | IVh |
|---|---|---|---|---|---|---|---|---|---|
| **IC**_{**50**}**/IC**_{**50CSA**} | 3.2 | 6 | 5.8 | 5.3 | 7.8 | 15.4 | 4 | 24.1 | 21.5 |

La courbe d'inhibition de l'activité cis/trans isomérase de la cyclophiline A par le dérivé IVb est représentée à la figure 5.

De manière surprenante, malgré les modifications importantes, par exemple des modifications stériques ou la fixations de la configuration des liaisons peptidiques, qu'apporte l'introduction d'une pseudo-proline dans la partie peptidique des cyclosporines A ou C qui est réputée se lier à la cyclophiline, il n'a pas été constaté de pertes importantes d'activité pour la plupart des dérivés, en particulier pour les dérivés IIb, IVa-d et IVf. On note même que des dérivés tels que le dérivé IVb dont la pseudo-proline porte un substituant à caractère hautement hydrophobe, un biphényl, inhibe relativement fortement la cyclophiline.

Comme on peut le voir, les dérivés de cyclosporine selon la présente invention possèdent des propriétés fort intéressantes.

L'introduction d'un pseudo-proline portant des substituants appropriés permet en particulier l'un ou plusieurs des effets suivants:
- amélioration des propriétés pharmacocinétiques de cyclosporines par solubilisation dans un milieu physiologique;
- obtention de prémédicaments ou "prodrug" de cyclosporines
- introduction de groupes réactifs permettant des "crosslinkings" ou des marquages;
- modulation de la conformation peptidique des cyclosporines du fait des contraintes stériques dues au cycle à cinq chaînons ce qui conduit à moduler l'activité biologiques des cyclosporines.

## Revendications

1. Dérivé de cyclosporine dont l'enchaînement peptidique comprend au moins un résidu d'acide aminé non naturel de formule générale I suivante: 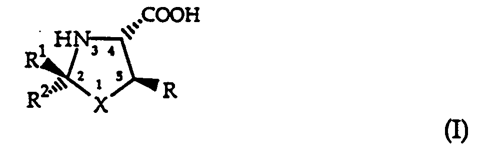 dans laquelle,
X représente un oxygène ou un soufre;
R représente un hydrogène ou un alkyle ayant de 1 à 6 atomes de carbone;
R₁ et R₂ représentent, indépendamment l'un de l'autre, un hydrogène, un alkyle ayant de 1 à 6 atomes de carbone, linéaire ou ramifié, substitué ou non, un alkylène ayant de 1 à 6 atomes de carbone, un aryle substitué ou non, un hétéro-aryle substitué ou non, un résidu d'un polymère hydrosoluble relié éventuellement à un groupe d'espacement.

2. Dérivé selon la revendication 1, **caractérisé en ce que**, dans l'acide aminé de formule générale I, R représente un hydrogène ou un méthyle.

3. Dérivé selon l'une des revendications précédentes, **caractérisé en ce qu'**il dérive d'une cyclosporine dont l'enchaînement peptidique contient au moins l'un des acides aminés choisi parmi sérine, thréonine ou cystéine, sous une configuration d ou 1.

4. Dérivé selon la revendication 3, **caractérisé en ce que** au moins l'un quelconque des acides aminés sérine, thréonine ou cystéine de cyclosporine de référence est remplacé par l'acide aminé de formule générale I.

5. Procédé de préparation des dérivés selon la revendication 4, comprenant une réaction de N,O-acétalisation d'au moins un des trois acides aminés sérine, thréonine ou cystéine en mettant en contact la cyclosporine de référence avec un composé de formule II suivante: 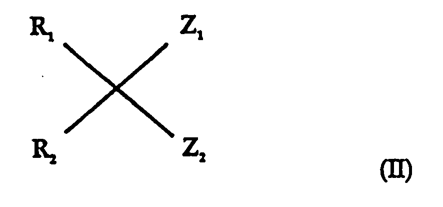 dans laquelle
Z₁ et Z₂ représentent, indépendamment l'un de l'autre, un halogène, un hydroxy, un alkoxy, un thiol; ou bien
Z₁ et Z₂ représentent ensemble, un oxygène d'un carbonyle ou un soufre d'une thione; et
R₁ et R₂ ayant la même définition que donnée précédemment.

## Claims

1. Cyclosporin derivative in which the peptide chain comprises at least one residue of a non-natural amino acid of general formula I: 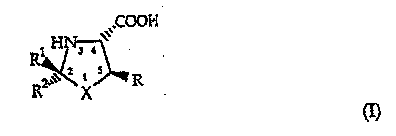 in which
X denotes an oxygen or a sulphur;
R denotes a hydrogen, or an alkyl group having between 1 and 6 carbon atoms;
R₁ and R² denote, independently of each other, a hydrogen, an alkyl group having between 1 and 6 carbon atoms, which may be straight-chain or branched-chain, substituted or non-substituted, an alkylene group having between 1 and 6 carbon atoms, a substituted or non-substituted aryl group, a substituted or non-substituted heteroaryl group, a residue of a water-soluble polymer optionally bonded to a spacer group.

2. Derivative according to Claim 1, **characterized in that** in the amino acid of general formula I, R denotes a hydrogen or a methyl group.

3. Derivative according to any one of the preceding claims, **characterized in that** it is derived from a cyclosporin in which the peptide chain contains at least one of the amino acids, chosen from serine, threonine and cysteine, in d or 1 configuration.

4. Derivative according to Claim 3, **characterized in that** at least any one of the amino acids serine, threonine or cysteine of the basic cyclosporin is replaced by the amino acid of general formula I.

5. Method of preparation of the derivatives according to Claim 4, comprising a reaction of N,O-acetalization of at least one of the three amino acids serine, threonine or cysteine by bringing the basic cyclosporin into contact with a compound of formula II: 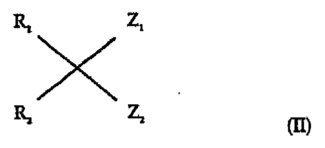 in which
Z₁ and Z₂ represent, independently of each other, a halogen, a hydroxyl group, an alkoxy group, a thiol; or both Z₁ and Z₂ represent either an oxygen of a carbonyl group or a sulphur of a thione; and R₁ and R₂ have the same definition as above.

## Patentansprüche

1. Cyclosporinderivat, das in seiner Peptidkette mindestens einen Rest einer nicht-natürlichen Aminosäure der folgenden allgemeinen Formel I: 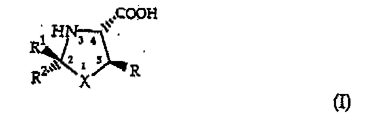 worin
X für Sauerstoff oder Schwefel steht;
R für Wasserstoff oder Alkyl mit 1 bis 6 Kohlenstoffatomen steht;
R₁ und R₂ anbhängig voneinander für Wasserstoff, gegebenenfalls substituiertes lineares oder verzweigtes Alkyl mit 1 bis 6 Kohlenstoffatomen, Alkylen mit 1 bis 6 Kohlenstoffatomen, gegebenenfalls substituiertes Aryl, gegebenenfalls substituiertes Heteroaryl oder einen Rest eines wasserlöslichen Polymers, der gegebenenfalls an eine Abstandhaltergruppe gebunden ist, stehen; enthält.

2. Derivat nach Anspruch 1, **dadurch gekennzeichnet, daß** in der Aminosäure der allgemeinen Formel I R für Wasserstoff oder Methyl steht.

3. Derivat nach einem der vorhergehenden Ansprüche, **dadurch gekennzeichnet, daß** es sich von einem Cyclosporin ableitet, das in seiner Peptidkette mindestens eine unter Serin, Threonin und Cystein in *D-* oder *L-*Konfiguration ausgewählte Aminosäure enthält.

4. Derivat nach Anspruch 3, **dadurch gekennzeichnet, daß** mindestens eine der Aminosäuren Serin, Threonin und Cystein des Ausgangs-Cyclosporins durch eine Aminosäure der allgemeinen Formel I ersetzt ist.

5. Verfahren zur Herstellung von Derivaten gemäß Anspruch 4, umfassend eine N,O-Acetalisierungsreaktion mindestens einer der drei Aminosäuren Serin, Threonin und Cystein durch Inberührungbringen des Ausgangs-Cyclosporins mit einer Verbindung der folgenden Formel II: 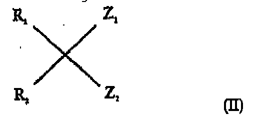 worin
Z₁ und Z₂ unabhängig voneinander für Halogen, Hydroxy, Alkoxy, Thiol stehen oder
Z₁ und Z₂ gemeinsam für Carbonylsauerstoff oder Thionschwefel stehen und
R₁ und R₂ die oben angegebene Bedeutung besitzen.
